Europäisches Patentamt

(19) European Patent Office

Office européen des brevets

(11) Publication number: **0 091 649**
A1

(12) # EUROPEAN PATENT APPLICATION

(21) Application number: **83103335.2**

(22) Date of filing: **06.04.83**

(51) Int. Cl.³: **A 23 K 1/17**

(30) Priority: **08.04.82 US 366519**

(43) Date of publication of application:
**19.10.83 Bulletin 83/42**

(84) Designated Contracting States:
**BE DE FR GB IT**

(71) Applicant: THE DOW CHEMICAL COMPANY
Dow Center 2030 Abbott Road Post Office Box 1967
Midland Michigan 48640(US)

(72) Inventor: Phillips, Dorothy Jean
5817 Sutton Place
Midland Michigan 48640(US)

(72) Inventor: Tadman, Jack Miron
5401 Gardenbrook
Midland Michigan 48640(US)

(74) Representative: De Carli, Elda et al,
PATENT DEPARTMENT c/o Gruppo Lepetit S.p.A. Via
Durando 38
I-20158 Milano(IT)

(54) Method for increasing the efficiency of rumen fermentation of ruminant animals.

(57) A method for improving the rumen fermentation in ruminant animals having a developed rumen function by administering an effective amount of the antibiotic Teichomycin $A_2$ or its physiologically acceptable salts, esters and amides. Increasing the efficiency of rumen fermentation is known to increase the efficiency of feed utilization and/or increase the rate of growth of ruminant animals.

EP 0 091 649 A1

## METHOD FOR INCREASING THE EFFICIENCY OF RUMEN FERMENTATION OF RUMINANT ANIMALS

### Background of the Invention

The metabolism of feed by ruminant animals such as cattle, sheep and goats having a developed rumen function has been a target of intensive investigation in recent years. It has been discovered that by improving the efficiency of rumen fermentation, a corresponding increase in the rate of growth and/or an increase in the efficiency of feed utilization by the animals will occur.

The overall efficiency of rumen fermentation is a function of the symbiotic activities of the microbial species inhabiting the rumen. These microbial species are responsible for the transformation of carbohydrates as well as protein and nonprotein nitrogenous substrates into forms such as microbial cell protein and volatile fatty acids suitable for biochemical utilization by the ruminant animal. The exact composition of the end products is a consequence of competition between the microbial species for the substrates or nutrients. The efficiency of the rumen fermentation is an important factor in determining

29,410-F

the degree of feed utilization efficiency and/or the rate of growth of the animal.

For example, during nitrogen metabolism, a portion of the feed protein (depending on the type of protein) is hydrolyzed by microbial enzymes in the rumen to ammonia and isoacids which are subsequently fixed into microbial protein. Other proteins from feed sources are metabolized to peptides and free amino acids. The peptides may subsequently be transformed into free amino acids by certain ruminal bacteria (for example, Bacteroides ruminicola) leaving a pool of free amino acids which may then be assimilated into microbial protein or catabolized to produce energy for microbial growth. The free amino acids may also be assimilated directly by the animal and used for protein synthesis or catabolized as a source of energy.

Carbohydrate metabolism provides energy for the growth of rumen microbes primarily through the fermentation of cellulose and starch. The insoluble polymers are converted to oligosaccharides and soluble sugars by extracellular enzymes from the rumen microorganisms. The resulting sugars are then fermented to one of various forms of volatile fatty acids, carbon dioxide and hydrogen. As used herein, the volatile fatty acids - acetic acid, propionic acid and butyric acid - are also referred to as acetate, propionate and butyrate, respectively. Volatile fatty acids are utilized by the animal as primary carbon and energy sources with varying degrees of efficiency. High levels of propionic acid are desirable because propionic acid is a primary metabolic precursor for gluconeogenesis in the animal. The fermentation of 6-carbon sugars to acetic acid is relatively inefficient since in this process, carbon is lost via eructation in the form of carbon dioxide or methane. On the other hand, the production of propionic acid does not result in a loss of carbon.

Rumen metabolism studies have shown that some of the rumen microbes such as various Ruminococcus and Butyrivibrio species ferment the monosaccharides of complex carbohydrates to formic, acetic, butyric and succinic acids, along with carbon dioxide and hydrogen. The carbon dioxide and hydrogen produced during fermentation are used in the formation of methane through the activity of methanogenic bacteria. Rumen microorganisms such as various Bacteroides species ferment carbohydrates predominantly to succinic acid which is converted to the desirable propionic acid by various Selenomonas species or other microorganisms.

It becomes possible then to improve feed utilization efficiency and/or the rate of growth of ruminant animals by selectively inhibiting or stimulating the growth of ruminal microflora involved in rumen fermentation. For instance, feed utilization efficiency and/or rate of growth can be improved by increasing the molar proportion of propionic acid to acetic acid or by increasing total volatile fatty acid concentration (i.e. the sum of acetic, propionic and butyric acids) in the rumen. For example, an increase in the molar portion of propionic acid can be accomplished by selectively inhibiting the growth of various species of Ruminococcus and Butyrivibrio and by stimulating the growth of various species of Bacteroides and Selenomonas. Likewise, the inhibition of methanogenic bacteria results in a shift of carbohydrate metabolism toward the production of the desirable, growth-improving volatile fatty acids, particularly propionic acid and butyric acid. Higher levels of the metabolic end products of feed protein degradation (such as ammonia and isoacids) can lead to such beneficial effects as a stimulation of microbial protein synthesis. In addition, a partial inhibition of the deamination activity of the rumen microflora makes more of the amino acids available for the nutrition of the animal.

Summary of the Invention

The present invention is directed to a method of improving the efficiency of rumen fermentation of ruminant animals having a developed rumen function. Improving the efficiency of rumen fermentation improves the efficiency of feed utilization and/or the rate of growth of ruminant animals. The novel method of the present invention requires the use of the antibiotic Teichomycin $A_2$, or its physiologically acceptable salts, esters and amides. As used herein, the phrase "Teichomycin $A_2$ or its physiologically acceptable salts, esters and amides" means at least one member selected from the group consisting of the antibiotic Teichomycin $A_2$, its physiologically acceptable salts, its physiologically acceptable esters and its physiologically acceptable amides. Thus, in the method and compositions described herein, Teichomycin $A_2$ or a physiologically acceptable salt, ester or amide thereof can be utilized individually or various combinations of Teichomycin $A_2$, its physiologically acceptable salts, esters or amides may be used. The antibiotic Teichomycin $A_2$ is disclosed and claimed in U.S. Patent 4,239,751 issued to Coronelli et al., December 16, 1980. The physiologically acceptable salts, physiologically acceptable esters and physiologically acceptable amides of Teichomycin $A_2$ are

readily prepared employing procedures well known in the art. Representative examples of the physiologically acceptable salts, esters and amides of Teichomycin $A_2$ are described below.

Teichomycin $A_2$ contains free carboxyl groups and thus exhibits the common property of an organic acid in that it forms salts. Representative of the inorganic bases forming physiologically acceptable cationic salts with Teichomycin $A_2$ are, for example, the alkali metal hydroxides such as lithium hydroxide, sodium hydroxide and potassium hydroxide; the alkali metal carbonates and bicarbonates such as lithium carbonate and sodium bicarbonate; the alkaline earth metal hydroxides and carbonates such as calcium hydroxide and magnesium carbonate; and like inorganic bases.

Illustrative of the organic bases forming physiologically acceptable salts with Teichomycin $A_2$ are the primary, secondary and tertiary $C_1-C_4$ lower alkyl and lower hydroxyalkyl amines such as ethylamine, isopropylamine, diethylamine, methyl-n-butylamine, ethanolamine, and diethanolamine.

The ammonium salts of Teichomycin $A_2$ are prepared with ammonia or various ammonium hydroxides.

Physiologically acceptable esters can readily be prepared by esterification of the carboxyl groups of Teichomycin $A_2$ to give esters such as alkyl esters, cycloalkyl esters and aryl esters. Typical alkyl esters are, for example, methyl, ethyl, isopropyl or butyl esters. Typical cycloalkyl esters are cyclopropyl and cyclohexyl esters. Typical of the aryl esters are those such as the phenyl ester.

Esters can also be made by acylation of one or more of the derivatives described herein having hydroxyl groups of these antibiotics. For example, esters are made by acylation using groups such as formyl, acetyl, cyclohexylacetyl, and benzoyl. Esters such as phosphate esters, can also be prepared from the hydroxyl groups of Teichomycin $A_2$.

Physiologically acceptable amide derivatives of Teichomycin $A_2$ can be prepared employing amines such as ethyl glycine, ethyl amine, diethylamine, n-methyl piperazine, or by employing acids such as acetic or succinic acid.

As used herein, the term "physiologically acceptable" when used in conjunction with salts, esters or amides refers to those salts, esters or amides of Teichomycin $A_2$ which will not cause significant adverse physiological effects when administered to an animal at dosages consistent with the method of the present invention. In the method and compositions described herein, Teichomycin $A_2$ or its physiologically acceptable salts, esters and amides are employed.

The antibiotic is administered in an amount sufficient to increase rumen fermentation efficiency which will provide an improvement in feed utilization efficiency and/or rate of growth in ruminant animals. For purposes of the invention, the amount of the antibiotic Teichomycin $A_2$ or its physiologically acceptable salts, esters and amides to be administered may range from 0.004 milligram (mg) per kilogram (kg) of ruminant animal body weight per day to 2 mg per kg of ruminant animal body weight per day, with a preferred range of from 0.01 mg per kg of ruminant animal body weight per day to 1 mg per kg of ruminant animal body weight per day. The exact amount of the antibiotic to be employed will vary depending upon factors such as the species of animal, or the size, weight, age, and health of the animal. In particular cases, the concentration to be administered may be determined by conventional dose titration techniques.

Teichomycin $A_2$ exerts a positive influence on metabolism of feed carbohydrate and protein in the rumen. The action of Teichomycin $A_2$ increases the availability of the nutrients contained in the feed, thus allowing improved efficiency of feed utilization by the animal. Teichomycin $A_2$ stimulates carbohydrate metabolism, increasing the rate of production of the major volatile fatty acids and increasing the energy available to the animal for growth. Teichomycin $A_2$ also stimulates the breakdown of feed protein in the rumen, resulting in an accumulation of amino acids, which are then available to the animal for increased protein synthesis. In the presence of Teichomycin $A_2$ the growth rate of the bacterial population is also stimulated, thus providing additional protein to the animal via normal digestion.

The effect of Teichomycin $A_2$ on the rumen fermentation is unique in contrast to commercial antibiotics. Antibiotics such as chlortetracycline, tylosin, oleandomycin, erythromycin, lincomycin and penicillin G cause a dramatic depression of metabolism in the rumen, which is observed as a decrease in the production of volatile fatty acids; the greatest depression is in the production of propionic acid, which is the major precursor for glucose synthesis in the animal. None of these commercial antibiotics exert positive effects on rumen metabolism which are beneficial to the animal.

Teichomycin $A_2$ is also unique in contrast to commercial ruminant feed additives such as monensin and lasalocid which are used to improve animal growth and feed utilization. The activity of these ionophores in the rumen is characterized by depression of microbial growth and feed protein breakdown. They also alter the ratio of volatile fatty acids produced in the rumen by the suppression of acetic acid production, thus increasing the molar proportion of propionic acid and decreasing the ratio of acetic acid to propionic acid. The overall effect of the ionophores in the rumen is the selective depression of microbial metabolism as compared to Teichomycin $A_2$ which stimulates microbial metabolism.

Stimulation of total volatile fatty acid production and increased availability of nitrogen may also lead to an increase in milk production in lactating ruminants thus benefiting industries such as the dairy industry.

Description of Some Preferred Embodiments

The method of the present invention involves the administration of the antibiotic Teichomycin $A_2$ or its physiologically acceptable salts, esters and amides to ruminant animals having a developed rumen function. The novel method of the present invention is equally applicable to all of the ruminant animals, however, the greatest impact from this invention should be realized in those industries involved with economically important ruminants such as cattle. For example, due to improved rumen fermentation, beef producing cattle treated in accordance with the method of the present invention will exhibit an improved rate of growth and/or will consume less feed per unit of weight gain.

The antibiotic Teichomycin $A_2$, or its physiologically acceptable salts, esters and amides may be incorporated into animal feed at a concentration of from 0.1 to 100 parts per million (ppm) by weight of the ultimate composition. Preferably, for ruminant animals, the antibiotic Teichomycin $A_2$, or its physiologically acceptable salts, esters and amides may be incorporated into ruminant animal feed at a concentration of from 0.1 to 80 ppm by weight of the ultimate composition, and especially preferred for ruminant animals in the range of from 0.2 to 40 ppm by weight of the ultimate composition. It may be added to drinking water or administered in the form of boluses, liquid feed compositions or incorporated into salt blocks. It may also be administered as an emulsion, suspension, tablet, capsule or any other appropriate veterinary dosage form in either single dose increments or in sustained release form. The incorporation of antibiotics into suitable dosage forms is well known in the pharmaceutical sciences. The method of this invention includes the incorporation of Teichomycin $A_2$ or its physiologically acceptable salts, esters, and amides into suitable dosage forms together with any additional diluents, adjuvants, excipients, fillers, stabilizers, disintegrators, matrixes, polymers, emulsifying and suspending agents or any other pharmaceutical vehicle or ingredients necessary to a finished dosage form.

For commercial use it is convenient to provide a feed additive premix, mineral supplement or concentrate containing the antibiotic Teichomycin $A_2$ or its physiologically acceptable salts, esters and amides in a proportion such that a predetermined quantity of the premix or concentrate may be added to a quantity of standard ruminant animal feed. The feed additive premix or concentrate comprises Teichomycin $A_2$ or its physiologically acceptable salts, esters and amides along with physiologically acceptable adjuvants and carriers such as soybean meal, ground corn, ground corn cobs, corn oil, barley, wheat or other edible feed grade material, mineral or vitamin mixtures, or an innocuous diluent such as an alcohol, a glycol or molasses which may be suitable for the particular animals being treated. For these purposes, the premix or concentrate may contain from 0.05 to 80 percent by weight and preferably of from 1 to 30 percent by weight of Teichomycin $A_2$ or its physiologically acceptable salts, esters and amides in admixture with a suitable adjuvant such as previously described. Crampton, et al. (Applied Animal Nutrition, 1969) and Church (Livestock Feeds and Feeding, 1977) further describe the process of admixing compounds such as Teichomycin $A_2$ into feed compositions, premixes and concentrates and are incorporated herein by reference.

The method of the present invention further contemplates treating a ruminant animal with one of the novel compositions containing Teichomycin $A_2$ or its physiologically acceptable salts, esters and amides in combination with one or more additives such as coccidiostats, antibiotics, minerals, vitamins or any other physiologically benefical agents employed in animal husbandry.

The following examples are set forth as a means of illustrating the present invention.  They are specific examples of preferred embodiments and are not intended as a limitation on the invention.

Example I

Two rumen-fistulated Holstein cows were tested over a period of five months which included pre-treatment, treatment, and post-treatment periods.  One of the cows was placed on a high grain, restricted roughage diet (hereinafter referred to as the hay-grain diet) and fed 7.2 kg per day of a mixture containing 1.8 kg of alfalfa hay and 5.4 kg of the composition shown below:

|  | (kg/100 kg of Total Composition) |
| --- | --- |
| Shelled corn, 86% dry matter | 71.553 |
| Corn gluten meal, 60% protein, 90% dry matter | 2.915 |
| Beet pulp, 90% dry matter | 21.381 |
| Dicalcium phosphate | 1.000 |
| Potassium sulfate | 0.673 |
| Calcium sulfate | 0.245 |
| Trace Mineral Salt[1] | 1.0 |
| Magnesium oxide | 0.096 |
| Vitamin premix[2] | + |
| Urea | 1.137 |
|  | 100.000 |
| percent dry matter | 87.47 |

[1]Commercially available Trace Mineral Salt for dairy cows was used.

[2]Commercially available premix was used to furnish 5511 International Units (IU) vitamin A and 2756 IU vitamin $D_3$ per kg dry matter of ration.

The other cow was placed on an all-roughage diet of alfalfa hay and trace mineral salt (hereinafter referred to as hay diet) and also fed 7.2 kg per day. Each diet contained at least 12 percent protein. The Teichomycin $A_2$ was added to the diets of each animal as a feed concentrate in a quantity representing the test concentration for a given treatment period. The Teichomycin $A_2$ was added to 0.45 kg of ground corn (hay-grain diet) or 0.45 kg of ground alfalfa hay (hay diet) at the concentration being tested in a particular treatment period and fed to the animals on a daily basis.

During the pre-treatment period, baseline analyses of the parameters to be measured during the investigation were made, and are set out below. The treatment periods consisted of three separate increments each 4 weeks long. During each increment a different concentration of the antibiotic was administered. During the first treatment period the antibiotic was administered to the cows at the rate of 5 mg per animal per day. In the second period, the rate was increased to 25 mg per animal per day, and finally to 100 mg of Teichomycin $A_2$ per animal per day in the third treatment period. Rumen fluid and blood samples were taken for analysis on days 7, 14, 21, and 28 of each treatment period. On each of the indicated days, the rumen fluid samples were taken just prior to feeding, at 30 minutes, 1 hour, and then at each hour with the last sample taken 8 hours after feeding. The pre-feeding rumen fluid samples were also used for weekly in vitro rumen fermentations. The blood samples were taken just prior to feeding and at 2 hour intervals, with the last sample taken 8 hours after feeding. A post-treatment period where no antibiotic was administered was utilized to evaluate return to pre-treatment values.

The blood samples were analyzed for blood urea nitrogen, glucose and ammonia. The rumen fluid samples were analyzed for volatile fatty acid concentrations. Protein degradation and amino acid deamination were monitored by measuring the levels of isoacids, (for example, iso-butyric, iso-valeric and valeric acids), alpha-amino nitrogen, and ammonia nitrogen. Microbial protein levels were also measured.

The data tabulated below clearly show the beneficial effects of the method of the present invention on *in vivo* rumen fermentation. The data also suggest that the method of this invention is not dependent upon the diet of the animal.

The numbers in each of the following tables are expressed as a percent of the pre-treatment or control values. Thus, a number greater than 100 indicates an increase in the indicated parameter as compared to the pre-treatment or control value, and a number smaller than 100 indicates a decrease in the indicated parameter as compared to the pre-treatment or control value.

Evaluation of Teichomycin $A_2$ in a
Fistulated Cow on a Hay Diet
(Results Expressed as Percent of the Pre-treatment Value[1])

| | Carbohydrate Metabolism | | | | | |
|---|---|---|---|---|---|---|
| | Acetate | Propionate | Butyrate | Total VFA[2] | A/P[3] | Total Isoacids |
| 5 mg/animal/day | 89* | 101 | 98 | 90* | 89 | 93 |
| 25 mg/animal/day | 146* | 178* | 174* | 154* | 88* | 153* |
| 100 mg/animal/day | 120* | 134* | 148* | 125* | 87* | 143* |
| Post-Treatment | 111* | 108 | 119* | 111* | 100 | 150* |

(Continued)

| | Nitrogen Metabolism | | | | | Blood (Plasma) | | |
|---|---|---|---|---|---|---|---|---|
| | | | Soluble | Insoluble Protein | | | | |
| | Ammonia-N | Amino-N | Protein | Bacterial | Protozoal | Glucose | Urea | Ammonia |
| 5 mg/animal/day | 123* | 94 | 83* | 116* | 95 | 97 | 60* | 76* |
| 25 mg/animal/day | 154* | 158* | 93 | 98 | 84* | 98 | 95 | 84* |
| 100 mg/animal/day | 203* | 213* | 108 | 178* | 105 | 116* | 106 | 92 |
| Post-Treatment | 263* | 221* | 120* | 161* | 125* | 104 | 136* | 80* |

[1]The means for the daily samples were averaged for each of the four weeks and these weekly means were then averaged to determine the 4 week mean. The 4 week mean is presented as a percent of the pre-treatment value.

[2]Total VFA = Total Volatile Fatty Acid (i.e., the sum of acetate, propionate and butyrate concentrations).

[3]A/P = Mole ratio of acetate to propionate.

* = Statistically significant at the 95 percent confidence level ($P<0.05$).

Evaluation of Teichomycin $A_2$ in
Fistulated Cow on a Hay-Grain Diet
(Results Expressed as Percent of the Pre-treatment Value[1])

| | Carbohydrate Metabolism | | | | | |
| | Acetate | Propionate | Butyrate | Total VFA[2] | A/P[3] | Total Isoacids |
|---|---|---|---|---|---|---|
| 5 mg/animal/day | 115* | 114* | 121* | 116* | 100 | 115* |
| 25 mg/animal/day | 125* | 119* | 133* | 125* | 104* | 115* |
| 100 mg/animal/day | 117* | 127* | 118* | 120* | 91* | 123* |
| Post-Treatment | 111* | 98 | 107 | 108 | 110* | 103 |

(Continued)

| | Nitrogen Metabolism | | | | | Blood (Plasma) | | |
|---|---|---|---|---|---|---|---|---|
| | | | Soluble | Insoluble Protein | | | | |
| | Ammonia-N | Amino-N | Protein | Bacterial | Protozoal | Glucose | Urea | Ammonia |
| 5 mg/animal/day | 134* | 229* | 119* | 118* | 115 | 100 | 104 | 75* |
| 25 mg/animal/day | 136* | 201* | 98 | 100 | 108 | 104 | 105 | 79* |
| 100 mg/animal/day | 144* | 288* | 131* | 80* | 122* | 118* | 94 | 92 |
| Post-Treatment | 153* | 219* | 133* | 114* | 133* | 106* | 99 | 71* |

[1]The means for the daily samples were averaged for each of the four weeks and these weekly means were then averaged to determine the 4 week mean. The 4 week mean is presented as a percent of the pre-treatment value.

[2]Total VFA = Total Volatile Fatty Acid (i.e., the sum of acetate, propionate and butyrate concentrations).

[3]A/P = Mole ratio of acetate to propionate.

* = Statistically significant at the 95 percent confidence level ($P < 0.05$).

In general, Teichomycin $A_2$ increased the concentration of total volatile fatty acids evidencing a general stimulation of carbohydrate metabolism and shifted the ratio of the acetate and propionate components toward the more efficient propionate form. Generally, nitrogen metabolism was also enhanced as noted by increased concentrations of isoacids, ammonia-nitrogen and amino-nitrogen as well as soluble protein. There was at least a partial inhibition of amino acid degradation as shown by significant increases in amino acid concentrations. In general, increased microbial protein levels were observed indicating a stimulation of microbial growth. It is believed that the inconsistent results seen only at the 5 mg/animal/day level in the animal on the hay diet were due to a diet formulation problem which was corrected during the indicated treatment period.

The in vitro studies were conducted in 24 hour batch fermentations in anaerobic digestors equipped with manometers. The manometers contained 20 percent NaCl acidified with HCl to a pH of less than 2 in order to measure the total gas production during the fermentation. The digestors contained 70 milliliters (ml) of fresh rumen fluid from one of the fistulated cows, the desired amount of antibiotic (i.e., 0, 0.2, 2.0 or 10 ppm), and 130 ml of a fermentation medium of the following composition:

| | |
|---|---|
| Mineral solution 1 | 7.5 ml |
| Mineral solution 2 | 7.5 ml |
| Micromineral solution | 1.5 ml |
| Resazurin solution 0.1% | 0.1 ml |
| Clarified rumen fluid | 10.0 ml |
| $NaHCO_3$ (6.33% solution) | 8.0 ml |
| $Na_2S \cdot 9H_2O$ (2.5% solution) | 0.5 ml |
| Distilled water | <u>64.9 ml</u> |
| | 100.0 ml |

To each 100 ml of the above preparation, 0.8 gram of dry nutrients were added. The dry nutrients consisted of 0.3 gram of Avicel[®] PH 101-microcrystalline cellulose, 0.3 gram of casein, and 0.1 gram each of anhydrous glucose and soluble starch.

The clarified rumen fluid of the fermentation medium was prepared by collecting rumen fluid from an untreated fistulated cow on a hay diet approximately 12 hours after feeding. The fluid was strained through gauze and centrifuged at 5,000 rpm. The supernatant was placed in one liter amber bottles (about 400 ml/-bottle) and autoclaved at 7 kg of pressure for sterilization.

All of the solutions of the fermentation medium were added as prepared stock solutions of the following compositions:

| Mineral Solution 1 | grams/liter |
|---|---|
| $K_2HPO_4$ | 12.5 |

| Mineral Solution 2 | |
|---|---|
| $KH_2PO_4$ | 12.5 |
| $MgSO_4 \cdot 7H_2O$ | 3.0 |
| NaCl | 12.0 |
| $CaCl_2 \cdot 2H_2O$ | 1.6 |

| Micro-Mineral Solution | |
|---|---|
| $FeSO_4 \cdot 7H_2O$ | 0.200 |
| $H_3BO_3$ | 0.030 |
| $CoCl_2 \cdot 6H_2O$ | 0.020 |
| $ZnSO_4 \cdot 7H_2O$ | 0.010 |
| $MnCl_2 \cdot 4H_2O$ | 0.003 |
| $Na_2MoO_4 \cdot 2H_2O$ | 0.003 |
| $NiCl_2 \cdot 6H_2O$ | 0.002 |
| $CuCl_2 \cdot 2H_2O$ | 0.001 |
| (Adjust pH to about 2) | |

| Resazurin Solution 0.1% | |
|---|---|
| Resazurin | 1.0 |

Sodium Bicarbonate Solution 6.33%

$NaHCO_3$     63.3
   (saturated with, and stored under 100% $CO_2$)


Sodium Sulfide Solution 2.5%

$Na_2S \cdot 9H_2O$     25.0
   (stored under nitrogen)


One 24 hour fermentation for each animal was carried out each week during all treatment periods. Gas composition analyses were made at 24 hours in order to determine the concentration of methane. The 24 hour methane values for each of the four weeks in a given treatment period were averaged. The fermentations to which Teichomycin $A_2$ had been added were compared to control fermentations (where no Teichomycin $A_2$ was added to the fermentation medium). Generally, from the data given below, it can be seen that methane production decreased when antibiotic levels in the fermentation medium were increased. The tables which follow summarize the data gained from fermentations of rumen fluid taken from the cow on the hay diet (Table I) and the cow on the hay-grain diet (Table II). The numbers represent the difference between the treated and control fermentations, expressed as a percent of control.

## TABLE I

### METHANE CONCENTRATION IN
### THE IN VITRO FERMENTATIONS - - HAY DIET
### (RESULTS EXPRESSED AS A PERCENT OF CONTROL)

| Treatment Period | Teichomycin $A_2$ Concentration in the In Vitro Fermentation Medium (ppm) | | |
|---|---|---|---|
| | 0.2 | 2 | 10 |
| Pre-Treatment | 105 | 88 | 78 |
| 5 mg/animal/day | 91 | 76 | 69 |
| 10 mg/animal/day | 91 | 80 | 71 |
| 25 mg/animal/day | 94 | 77 | 59 |
| Post-Treatment | 95 | 81 | 73 |

## TABLE II

### METHANE CONCENTRATION IN
### THE IN VITRO FERMENTATIONS -- HAY-GRAIN DIET
### (RESULTS EXPRESSED AS A PERCENT OF CONTROL)

| Treatment Period | Teichomycin $A_2$ Concentration in the In Vitro Fermentation Medium (ppm) | | |
|---|---|---|---|
| | 0.2 | 2 | 10 |
| Pre-Treatment | 97 | 92 | 81 |
| 5 mg/animal/day | 102 | 84 | 68 |
| 10 mg/animal/day | 100 | 98 | 88 |
| 25 mg/animal/day | 105 | 99 | 87 |
| Post-Treatment | 94 | 89 | 82 |

## Example II

In *vitro* evaluations of the antibiotic Teichomycin $A_2$ were carried out in 24 hour batch fermentations in anaerobic digestors having gas and liquid sampling ports and manometers to measure total gas production during the fermentation. Three different concentrations of Teichomycin $A_2$ (0.2, 1.0 and 5.0 ppm) were prepared in 5 percent methanol solutions and placed in three separate groups of digestors. Fresh rumen fluid (700 ml) from an untreated fistulated cow was added to 1300 ml of the fermentation medium described in Example I and mixed. After mixing, 10 ml were removed and analyzed as a control, and 200 ml were placed in each of the digestors. The manometers were attached and nitrogen was bubbled through to remove oxygen. The digestors were then placed in a 40°C water bath and continuously agitated. The pH of the medium was checked and adjusted to pH 6.8-7.2 with $CO_2$.

The cultures were sampled at 0, 5 and 24 hours. The 5 hour sample was used primarily to note effects on nitrogen metabolism. Measurement of the change in concentration of protein, amino acids and ammonia in the fermentation after 5 hours was an indication of the extent to which the rate of protein degradation and deamination were inhibited by the antibiotic being tested. Concentrations of isoacids, i.e. iso-butyric, iso-valeric and valeric acids were determined at 24 hours and used as a measure of inhibition of deamination since the major source of these acids is the deamination of the amino acids valine, leucine and proline, respectively.

The 24 hour sample was used to measure volatile fatty acid production. The mole ratio of acetate to propionate (A/P) was used to determine if the antibiotic increased the molar proportion of propionate in total volatile fatty acid concentrations. The gas composition was also determined at 24 hours and the amount of methane produced was noted. The rate at which gas was produced was determined by reading the manometers hourly at 3, 4 and 5 hours to monitor microbial metabolism. The results of these in vitro fermentations are set forth below, and are expressed as a percent of control. The data generally show that Teichomycin $A_2$ improved rumen fermentation efficiency as evidenced by an increase in the molar proportion of propionate (a decreased A/P ratio). The data also show that Teichomycin $A_2$ inhibited deamination as evidenced by an increase in amino nitrogen concentration and a decrease in isoacid concentration.

In Vitro Evaluation of Teichomycin A$_2$
(expressed as a percent of control)[2]

| Concentration | Gas Rate | Methane | Protein Degradation | Amino-N | Ammonia-N |
|---|---|---|---|---|---|
| 0.2 ppm[1] | 96 | 100 | 92 | 90 | 108 |
| 1 ppm[1] | 103 | 102 | 94 | 110 | 108 |
| 5 ppm[1] | 98 | 94 | 90 | 153 | 73 |

(Continued)

| Isoacids | Acetate | Propionate | Butyrate | Total VFA[2] | A/P[3] |
|---|---|---|---|---|---|
| 120 | 83 | 102 | 116 | 93 | 81 |
| 94 | 89 | 109 | 112 | 98 | 83 |
| 62 | 81 | 122 | 120 | 93 | 67 |

[1]Average of 3 batch fermentations.

[2]Total VFA = Total Volatile Fatty Acid (i.e., the sum of acetate, propionate and butyrate concentrations).

[3]A/P = Mole ratio of acetate to propionate.

29,410-F

Example III

To show that the method of the present invention is not limited by the type of ruminant animal treated, the following study was conducted using sheep rumen fluid. In _vitro_ fermentations were performed following substantially the same procedures as described in Example II. The anaerobic digestors contained sheep rumen fluid and Teichomycin $A_2$ in a concentration of 5 ppm. The data are set forth below and are expressed as a percent of control. An improvement in rumen fermentation was evidenced by an increase in the molar proportion of propionate and a decrease in the production of methane. Total isoacids were depressed and amino-nitrogen levels were increased indicating an inhibition of deamination.

## In <u>Vitro</u> Evaluation of Teichomycin $A_2$
### in Sheep Rumen Fluid
### (expressed as a percent of control)

| Concentration | Gas Rate | Methane | Protein Degradation | Amino-N | Ammonia-N |
|---|---|---|---|---|---|
| 5 ppm | 118 | 74 | 98 | 123 | 75 |

(Continued)

| Isoacids | Acetate | Propionate | Butyrate | Total VFA.[1] | A/P[2] |
|---|---|---|---|---|---|
| 47 | 86 | 117 | 90 | 96 | 74 |

[1]Total VFA = Total Volatile Fatty Acid (i.e., the sum of acetate, propionate and butyrate levels).

[2]A/P = Mole ratio of acetate to propionate.

WHAT IS CLAIMED IS:

1.   A process for improving the efficiency
of rumen fermentation in ruminant animals having a
developed rumen function which comprises administering
to said animals the antibiotic Teichomycin $A_2$ or its
physiologically acceptable salts, esters and amides.

2.   The process of Claim 1 wherein the
amount of the antibiotic Teichomycin $A_2$ or its
physiologically acceptable salts, esters and
amides administered is from 0.004 mg per kg of
ruminant animal body weight per day to 2 mg per
kg of ruminant animal body weight per day.

3.   The process of Claim 2 wherein the
amount of the antibiotic Teichomycin $A_2$ or its physio-
logically acceptable salts, esters and amides
administered is from 0.01 mg per kg of ruminant
animal body weight per day to 1 mg per kg of ruminant
animal body weight per day.

4.   A process for improving the efficiency
of rumen fermentation in ruminant animals having a
developed rumen function which comprises administering to said animals a ruminant animal feed composition containing from 0.1 to 80 parts per million by weight of the ultimate composition of the antibiotic Teichomycin $A_2$.

5. The process of Claims 1, 2, 3, or 4 wherein the ruminant animals are cattle.

6. The process of Claims 1, 2, 3, or 4 wherein the ruminant animals are sheep.

7. The process of Claims 1, 2, or 3 wherein the antibiotic Teichomycin $A_2$ or its physiologically acceptable salts, esters and amides is administered in admixture with ruminant animal feed.

8. The process of Claim 7 wherein the ruminant animals are cattle.

9. The process of Claim 7 wherein the ruminant animals are sheep.

10. An animal feed composition which comprises a mixture of animal feed and the antibiotic Teichomycin $A_2$ or its physiologically acceptable salts, esters and amides.

11. The animal feed composition of Claim 10 wherein the antibiotic Teichomycin $A_2$ or its physiologically acceptable salts, esters and amides is present in a concentration of from 0.1 to 100 parts per million by weight of the ultimate composition.

12. A ruminant animal feed composition suitable for improving the efficiency of rumen fermentation in ruminant animals having a developed rumen function which comprises a mixture of ruminant animal feed and the antibiotic Teichomycin $A_2$ or its physiologically acceptable salts, esters and amides.

13.     The ruminant animal feed composition of Claim 12 wherein the antibiotic Teichomycin $A_2$ or its physiologically acceptable salts, esters and amides is present in a concentration of from 0.1 to 80 parts per million by weight of the ultimate composition.

14.     The ruminant animal feed composition of Claim 13 wherein the antibiotic Teichomycin $A_2$ or its physiologically acceptable salts, esters and amides is present in a concentration of from 0.2 to 40 parts per million by weight of the ultimate composition.

15.     A ruminant animal feed concentrate comprising the antibiotic Teichomycin $A_2$ or its physiologically acceptable salts, esters and amides.

16.     The ruminant animal feed concentrate of Claim 15 whereby said concentrate is mixed with a standard ruminant animal feed.

17.     The ruminant animal feed concentrate of Claim 15 wherein the antibiotic Teichomycin $A_2$ or its physiologically acceptable salts, esters and amides is present in a concentration of from 0.05 to 80 percent by weight of the ultimate concentrate.

18.     The ruminant animal feed concentrate of Claim 17 wherein the antibiotic Teichomycin $A_2$ or its physiologically acceptable salts, esters and amides is present in a concentration of from 1 to 30 percent by weight of the ultimate concentrate.

0091649

European Patent Office

**EUROPEAN SEARCH REPORT**

Application number

EP 83 10 3335

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,A | US-A-4 239 751 (C. CORONELLI et al.) <br> * Claim 1 * | | A 23 K 1/17 |
| A | GB-A-2 057 873 (BRISTOL-MYERS CO.) <br> * Abstract * | | |

TECHNICAL FIELDS SEARCHED (Int. Cl. ³)

A 23 K 1/00
C 07 G 11/00

The present search report has been drawn up for all claims

| Place of search <br> BERLIN | Date of completion of the search <br> 27-06-1983 | Examiner <br> SCHULTZE D |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82